# EUROPEAN PATENT APPLICATION

(11) **EP 4 541 909 A1**
(43) Date of publication of application: **23.04.2025**
(21) Application number: 22946955.6
(22) Date of filing: 17.06.2022
(51) Int. Cl.: C12Q 1/6886, G01N 33/574

(54) **MARKER FOR PROGNOSIS OF DIFFUSE TYPE GASTRIC CANCER AND TREATMENT TARGET**

(71) Applicant: Korea Research Institute of Bioscience and Biotechnology, Daejeon 34141 (KR); Industry-Academic Cooperation Foundation, Yonsei University, Seoul 03722 (KR)
(72) Inventor: CHEONG, Jae-Ho, Seoul 03722 (KR); WON, Mi Sun, Daejeon 34141 (KR); KIM, Bo Kyung, Daejeon 34141 (KR); PARK, Kyung Chan, Daejeon 34141 (KR)
(74) Representative: Jung, Minkyu
(86) International application number: PCT/KR2022/008581
(87) International publication number: WO 2023/243749

(57) **Abstract**

The present disclosure provides a composition and a kit for diagnosis or prognosis of diffuse type gastric cancer, each comprising an agent for measuring an expression level of *SYT11* gene. Also provided in the present disclosure is a method for providing information necessary for the diagnosis of diffuse type gastric cancer or for determining a therapeutic strategy for diffuse type gastric cancer.

## Description

### [Technical Field]

The present disclosure relates to a composition for diagnosis or prognosis of diffuse type gastric cancer capable of diagnosing diffuse type gastric cancer or predicting the prognosis of diffuse type gastric cancer, a kit including the same, and a method for providing information necessary for the diagnosis of diffuse type gastric cancer or for determining a therapeutic strategy for diffuse type gastric cancer.

### [Background Art]

Gastric cancer is cancer with the top incidence rate of the world in Korea, and is one of diseases having a great need for prevention, treatment, and prognosis therefor. The gastric cancer may be divided into an intestinal type and a diffuse type classified according to the Lauren classification based on pathology and histology. The diffuse type gastric cancer is characterized by proliferating in a spreading manner under the gastric mucosa and allowing cancer cells to be infiltrated into the gastric wall. However, the diffuse type gastric cancer has a worse survival rate than intestinal type gastric cancer, and is particularly difficult to be detected in the early stages because the incidence is high among young people in 20s and 30s who have relatively little awareness of cancer or opportunities for health checkups. Therefore, the diffuse type gastric cancer has a greater need to detect the onset of gastric cancer at an early stage, and there is a growing need for research on methods for diagnosing diffuse type gastric cancer even in gastric cancer in order to establish a therapeutic strategy.

Up until now, most of testing methods for diagnosing gastric cancer have involved physical means such as X-ray radiography or gastroscopy, but these methods have a disadvantage of being painful for patients to be tested and requiring a cumbersome process. Accordingly, research has been conducted on genetic markers that are specifically expressed or have increased expression levels in gastric cancer patients, and research is continuing to measure the expression levels of gastric cancer markers to be used for diagnosis. However, research on genetic markers related to diagnosis of diffuse type gastric cancer according to histological classification even in gastric cancer, or research related to the prognosis of patients with diffuse type gastric cancer is insufficient.

Meanwhile, the research team of the present disclosure has proposed research using an *SYT11* gene as a diagnostic marker for 'stem-like gastric cancer' through Korean Patent Publication No. 2020-0044695 relating to a composition for treating gastric cancer including a Synaptotagmin 11 (*SYT11*) inhibitor as an active ingredient, and a method for diagnosing stem-like gastric cancer including measuring the expression of *SYT11.* However, a relation between diffuse type gastric cancer with different characteristics therefrom and the expression level of the *SYT11* gene has not been reported. In addition, when administering a therapeutic agent targeting *SYT11,* such as the *SYT11* inhibitor, no research has been conducted on marker genes that may be used for companion diagnostics to predict the effect of a therapeutic agent in advance and establish a therapeutic strategy suitable for a specific patient. Therefore, there is a need to develop a novel marker that may be used to diagnose diffuse type gastric cancer, predict its prognosis, and establish a therapeutic strategy such as application of a *SYT11* targeted therapeutic agent, and a method for providing information necessary for the diagnosis of diffuse type gastric cancer or for determining a therapeutic strategy for diffuse type gastric cancer through the same.

### [Disclosure]

### [Technical Problem]

An object of the present disclosure is to provide a composition and a kit capable of diagnosing diffuse type gastric cancer even in gastric cancer or predicting prognosis of patients with diffuse type gastric cancer.

In addition, another object of the present disclosure is to provide a method for providing information necessary for the diagnosis of diffuse type gastric cancer or for determining a therapeutic strategy for patients with diffuse type gastric cancer.

### [Technical Solution]

In order to achieve the object, an aspect of the present disclosure provides a composition for diagnosis or prognosis of diffuse type gastric cancer, including an agent for measuring an expression level of *SYT11* gene.

In addition, another aspect of the present disclosure provides a kit for diagnosis or prognosis of diffuse type gastric cancer, including the composition.

In addition, yet another aspect of the present disclosure provides a method for providing information necessary for the diagnosis of diffuse type gastric cancer or for determining a therapeutic strategy for diffuse type gastric cancer, including measuring an expression level of *SYT11* gene from an isolated biological stomach tissue sample, in which the information necessary for determining the therapeutic strategy for diffuse type gastric cancer is at least one selected from the group consisting of predicting the prognosis of diffuse type gastric cancer; evaluating the prognosis of diffuse type gastric cancer; and evaluating the efficacy of a *SYT11* targeted therapeutic agent in gastric cancer patients.

### [Advantageous Effects]

According to the present disclosure, it was confirmed that the expression level of *SYT11* gene showed a higher correlation with patients with diffuse type gastric cancer than intestinal type gastric cancer, and also had a high relation with the prognosis of the patients with diffuse type gastric cancer, and thus the *SYT11* gene can be usefully used as a marker for the diagnosis or prognosis of diffuse type gastric cancer. Further, it was confirmed that the expression levels of eight genes other than the *SYT11* gene had similar patterns to the expression level of the *SYT11* gene in patients with diffuse type gastric cancer to be closely related to the prognosis of the patients with diffuse type gastric cancer. Therefore, the eight genes can be usefully used together with the *SYT11* gene in the diagnosis or establishing a therapeutic strategy for diffuse type gastric cancer.

However, the effects of the present disclosure are not limited to the above-mentioned effects, and other effects not mentioned will be clearly understood by those skilled in the art from the following description.

### [Description of Drawings]

FIG. 1 is a photograph showing normal stomach tissue, intestinal type gastric cancer tissue, and diffuse type gastric cancer tissue confirmed through a microscope, and a table classified into groups with low (*SY711*-low) or high (*SY711*-high) expression levels of *SYT11* gene based on data on the expression level of *SYT11* gene measured for each of the tissues.
FIG. 2 shows results of classifying 424 gastric cancer patients into intestinal type or diffuse type gastric cancer based on clinical data, and then classifying the gastric cancer patients again according to a difference between the expression level of *SYT11* gene and the presence of stem-like gastric cancer.
FIG. 3 is a graph comparing survival probability according to a type of gastric cancer (intestinal type or diffuse type gastric cancer) and an expression level of *SYT11* gene (FIGS. 3A and 3B) or *SYT4* gene (FIGS. 3C and 3D) based on clinical data of 424 gastric cancer patients.
FIG. 4 is a table and graphs comparing and analyzing patterns of the expression levels of *SYT11* gene and eight genes (*ANGPTL2, CACNB1, CCL19, CD34, JAM3, SLIT2, THBS4,* and *VIMENTIN* genes) in patients with diffuse type gastric cancer.
FIG. 5 is a graph analyzing and comparing survival probability according to a type of gastric cancer (intestinal type or diffuse type gastric cancer) and expression levels of *SYT11* gene and eight genes (*ANGPTL2, CACNB1, CCL19, CD34, JAM3, SLIT2, THBS4,* and *VIMENTIN* genes)*,* based on clinical data of gastric cancer patients.
FIG. 6 shows results of performing Western blotting for SYT11 protein and ANGPTL2, THBS4, VIMENTIN, and JAM3 proteins after transfecting SNU484 cells as a gastric cancer cell line with siSYT11 capable of functioning as a SYT11-targeting therapeutic agent, and a control group siScramble (siSC).
FIG. 7 shows results of performing Western blotting for SYT11 protein and ANGPTL2, THBS4 and JAM3 proteins after transfecting SNU484 cells as a gastric cancer cell line with SYT11-ASO capable of functioning as a SYT11-targeting therapeutic agent, and a control group NC-ASO.

### [Modes of the Invention]

First, the terms used in the present disclosure are defined.

In the present disclosure, "prognosis" means a forecast of the future medical outcome (e.g., overall survival rate, recurrence-free survival rate, etc.) of a patient's disease, and includes positive prognosis including the remission of disease (e.g., recovery of disease, etc.), the improvement of disease (e.g., regression of tumor, etc.), or the stabilization of disease, and negative prognosis including the recurrence of disease, and the progression or fatality of disease (e.g., tumor growth, metastasis, drug resistance, etc.).

In the present disclosure, "prediction" means guessing in advance the future medical outcome of a patient's disease, and includes prediction of the course of the patient's disease (e.g., the remission, improvement, stabilization, recurrence, progression, fatality, deterioration, etc. of disease).

In the present disclosure, a "biological sample" means a tissue obtained from a patient with diffuse type gastric cancer or a normal subject, and specifically may be stomach tissue.

Hereinafter, the present disclosure will be described in detail.

### 1. Composition and kit for diagnosis or prognosis of diffuse type gastric cancer

The present disclosure provides a composition for diagnosing diffuse type gastric cancer or predicting the prognosis of diffuse type gastric cancer.

The composition for the diagnosis or prognosis of diffuse type gastric cancer includes an agent for measuring an expression level of *SYT11* gene.

The diffuse type gastric cancer is distinguished from intentional type gastric cancer when classifying gastric cancer according to the Lauren classification based on pathological and histological characteristics, and is gastric cancer that exhibits the characteristic of infiltrating individual cancer cells into the gastric wall without forming a distinct mass due to low adhesion of cancer cells. The intestinal type gastric cancer is gastric cancer in which cancer cells gather in one location and grow in a lump shape.

The *SYT11* gene (Synaptotagmin 11, ILMN_1717934) is a type of the Synaptotagmin gene family and encodes a protein similar to other family member proteins known as calcium sensors. The protein expressed from the *SYT11* gene may serve to coordinate calcium-dependent regulation of membrane transport during a synaptic transmission process. A base sequence of the *SYT11* gene and an amino acid sequence of the protein expressed from the *SYT11* gene may be obtained from public database such as NCBI's GenBank or from literatures.

The composition for the diagnosis or prognosis of diffuse type gastric cancer may further include at least one agent for measuring an expression level of a gene selected from the group consisting of *ANGPTL2, CACNB1, CCL19, CD34, JAM3, SLIT2, THBS4,* and *VIMENTIN.*

The base sequences of the *ANGPTL2* gene (Angiopoietin-like protein 2, ILMN_1772612), *CACNB1* gene (Calcium Voltage-Gated Channel Auxiliary Subunit Beta 1, ILMN_2271149), *CCL19* gene (C-C Motif Chemokine Ligand 19, ILMN_1769129), *CD34* gene (ILMN_1732799), *JAM3* gene (Junctional adhesion molecule C, ILMN_1769575), *SLIT2* gene (Slit homolog 2 protein, ILMN_1676449), *THBS4* gene (Thrombospondin-4, ILMN_1736078), and *VIMENTIN* gene (ILMN_1782538) and the amino acid sequences of the proteins expressed from the genes may be obtained in public database such as NCBI's GenBank or in the literatures.

The agent for measuring the expression level of the gene may be an agent for measuring the amount of mRNA of the gene or the amount of protein expressed from the gene.

The agent for measuring the amount of mRNA of the gene means an agent capable of specifically binding to and recognize mRNA of the gene or amplify the amount, and includes a primer pair or probe that specifically binds to a base sequence of the mRNA or cDNA produced by reverse transcription of the mRNA.

The primer is a short nucleic acid sequence having a free 3' hydroxyl group, which forms base pairs with a complementary template strand, and a nucleic acid sequence that serves to provide a starting point for nucleic acid polymerase to replicate and amplify the template strand. In addition, the probe means a nucleic acid fragment having a length of several bases to several hundred bases and consisting of a sequence capable of forming a specific binding to mRNA or cDNA.

The amount of mRNA of the gene may be measured by performing methods such as PCR, RT-PCR, competitive RT-PCR, real-time RT-PCR, and the like using sense and antisense primers of the gene sequence. In addition, the amount of mRNA of the gene may be measured by performing methods such as Northern blot, microarray, and the like using a probe having a sequence capable of specifically binding to mRNA of the gene or cDNA produced by reverse transcription of the gene. Further, the amount of mRNA of the gene may be measured by methods such as RNase protection assay, sequencing, and the like, but is not limited thereto and any agent necessary for using any method known to those skilled in the art may be used.

The agent for measuring the amount of the protein expressed from the gene means an agent capable of specifically binding to and recognizing the protein, and includes an antibody or aptamer that specifically binds to the protein.

The antibody refers to an immunoglobulin molecule that immunologically binds specifically to an epitope of the protein to have reactivity, and may include all a monoclonal antibody, a polyclonal antibody, an antibody having a full-length chain structure, an antibody of a functional fragment having at least an antigen-binding function, and a recombinant antibody. The aptamer refers to a single-stranded nucleic acid molecule that has the characteristic capable of forming a specific binding targeting the protein and forms a stable three-dimensional structure, and an aptamer having specificity for the protein may be synthesized using Systematic Evolution of Ligands by Exponential enrichment (SELEX) technology, etc.

The amount of protein expressed from the gene may be measured using the antibody or aptamer by a method such as Western blot, protein microarray (protein chip), Enzyme Linked Immunosorbent Assay (ELISA), 2-dimentional electrophoresis, Immunohistochemistry (IHC), immunofluorescence, co-immunoprecipitation assay, Fluorescence activated cell sorter (FACS), radioimmunoassay (RIA), radioimmunodiffusion, Matrix Assisted Laser Desorption/Ionization Time of Flight Mass Spectrometry (MALDI-TOF analysis), etc., but is not limited thereto and any agent necessary for using any method known to those skilled in the art may be used.

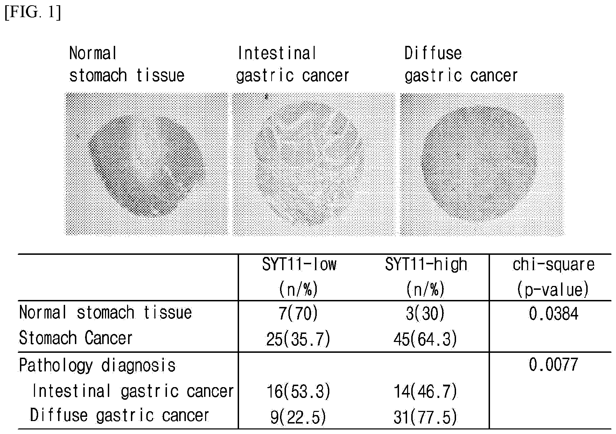

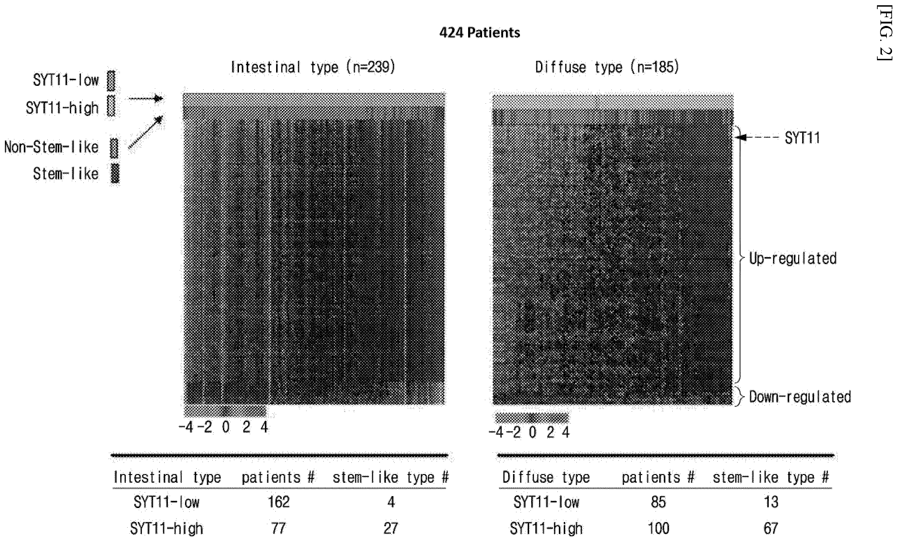

The composition including the agent for measuring the expression level of the *SYT11* gene may be used for providing information necessary for the diagnosis of diffuse type gastric cancer. In a specific embodiment of the present disclosure, it was confirmed that the expression level of the *SYT11* gene was high in patients with diffuse type gastric cancer, unlike intestinal type gastric cancer. Therefore, the composition of the present disclosure may be used to provide information for diagnosing diffuse type gastric cancer when the expression level of the *SYT11* gene is high by measuring the expression level of the *SYT11* gene for patients whose occurrence of gastric cancer is unknown or patients whose histological classification is unknown (FIGS. 1 and 2).

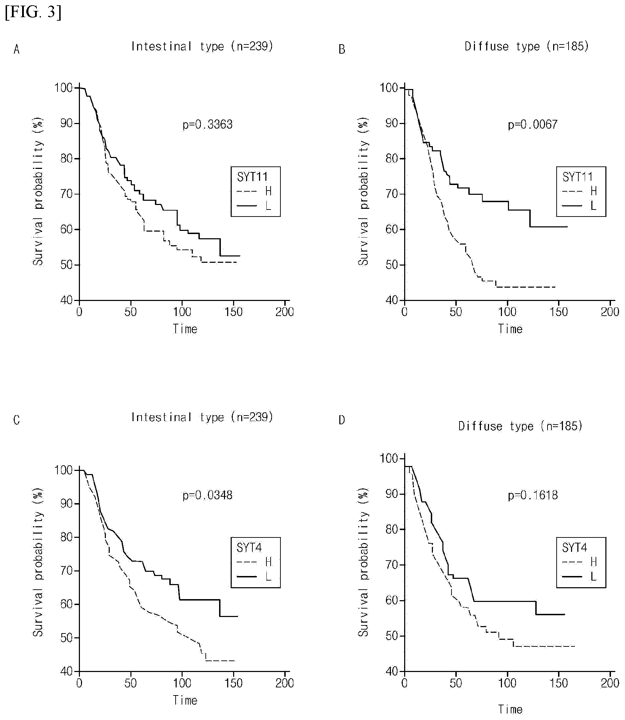

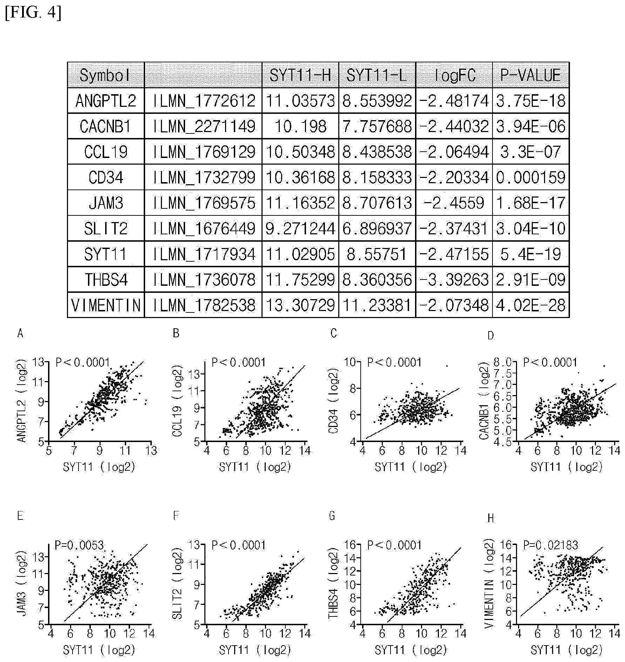

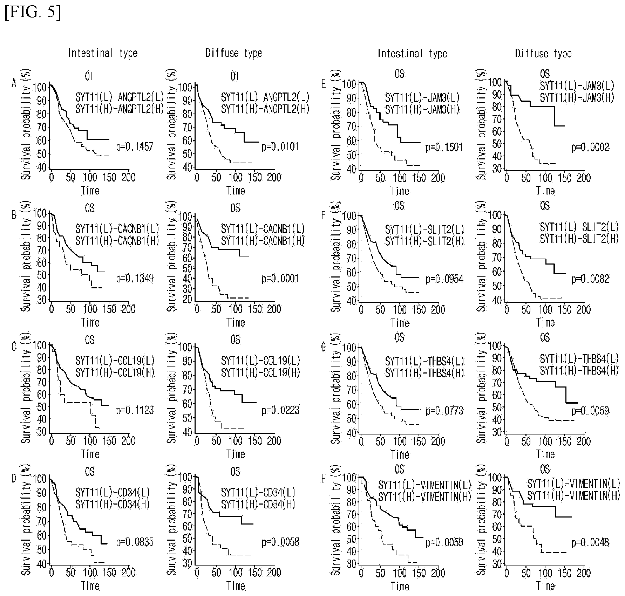

In addition, a composition including the agent for measuring the expression level of the *SYT11* gene or further including at least one agent for measuring the expression level of a gene selected from the group consisting of *ANGPTL2, CACNB1, CCL19, CD34, JAM3, SLIT2, THBS4*and *VIMENTIN* is able to be used for predicting the prognosis of patients with diffuse type gastric cancer. As a result of measuring the expression level of the *SYT11* gene and the expression level of at least one gene selected from the group consisting of *ANGPTL2, CACNB1, CCL19, CD34, JAM3, SLIT2, THBS4,* and *VIMENTIN,* it is possible to predict that the prognosis of patients with diffuse type gastric cancer will be poor when the expression level of the gene is high, thereby establishing strategies and measures for treatment. In this regard, in a specific embodiment of the present disclosure, it was confirmed that the expression level of the *SYT11* gene was higher in patients with diffuse type gastric cancer even among gastric cancer patients, and it was confirmed that the survival probability of patients with diffuse type gastric cancer having a high expression level of the *SYT11* gene was lower than that of patients with diffuse type gastric cancer having a low expression level of the *SYT11* gene (FIG. 3). In addition, in the patients with diffuse type gastric cancer having the high expression level of the *SYT11* gene, the expression levels of the *ANGPTL2, CACNB1, CCL19, CD34, JAM3, SLIT2, THBS4,* and *VIMENTIN* genes were also measured to be high (FIG. 4), and it was confirmed that the expression levels of the genes were also related to the survival probability of patients with diffuse type gastric cancer (FIG. 5).

The present disclosure provides a kit for diagnosing diffuse type gastric cancer or predicting the prognosis of diffuse type gastric cancer.

The kit includes the composition for the diagnosis or prognosis of the diffuse type gastric cancer.

The kit may include a composition including the agent for measuring the expression level of the *SYT11* gene, or a composition including an agent for measuring the expression level of the *SYT11* gene, and at least one agent for measuring the expression level of a gene selected from the group consisting of *ANGPTL2, CACNB1, CCL19, CD34, JAM3, SLIT2, THBS4,* and *VIMENTIN.*

The kit may further include other ingredients suitable for measuring the amount of mRNA present in the genes. For example, the kit may be in the form of a RT-PCR kit and a DNA chip kit, or may further include components of the RT-PCR kit and the DNA chip kit, and any kit in the form to be used for measuring the amount of mRNA known to those skilled in the art may be applied to the kit of the present disclosure.

Specifically, the kit may be a kit including essential components required for performing RT-PCR. For example, in addition to a primer pair capable of specifically binding to the mRNA of the gene, the kit may include a test tube or other appropriate containers, an enzyme such as Taq polymerase and reverse transcriptase, a reaction buffer (having various pH and magnesium concentrations), deoxynucleotide (dNTP), DNase and RNase inhibitors, DEPC water, sterile water, and the like. In addition, the kit may include a primer pair capable of specifically binding to a gene used as a control group during quantitation.

In addition, the kit may be a kit including essential components required to perform a DNA chip. The kit may include a glass substrate to which an oligonucleotide capable of specifically binding to mRNA or reverse transcribed cDNA of the gene is attached, and reagents, enzymes, agents, etc. for producing a fluorescently labeled probe.

The kit may include a composition, solution or device including one or more other ingredients suitable for measuring the amount of proteins expressed from the genes. For example, the kit may be in the form of an ELISA kit and a protein chip kit, or may further include components of the ELISA kit and the protein chip kit, and any kit in the form to be used for measuring the amount of protein known to those skilled in the art may be applied to the kit of the present disclosure.

Specifically, the kit may be a kit including essential components required to perform ELISA. For example, the kit may include a substrate for immunological detection of an antibody, a suitable reaction buffer, and a reagent capable of detecting the formation of an antigen-antibody complex using an antibody capable of specifically binding to a protein expressed from the gene, and may include an enzyme mediating a color reaction, a secondary antibody labeled with a fluorescent substance, etc., a color development substrate, and the like. Examples of the enzyme mediating the color reaction may include alkaline phosphatase, acid phosphatase, and peroxidase, and examples of the fluorescent substance may include colloid gold, fluorescein carboxylic acid (FCA), fluorescein isothiocyanate (FITC), rhodamine-B-isothiocyanate (RITC), fluorescein thiourea (FTH), 7-acetoxycoumarin-3-yl, fluorescein-5-yl, fluorescein-6-yl, 2',7'-dichlorofluorescein-5-yl, 2',7'-dichlorofluorescein-6-yl, dihydrotetramethylrosamine-4-yl, tetramethylrhodamine-5-yl, tetramethylrhodamine-6-yl, 4,4-difluoro-5,7-dimethyl-4-bora-3a,4a-diaza-indacene-3-ethyl, 4,4-difluoro-5,7-diphenyl-4-bora-3a,4a-diaza-s-indacene-3-ethyl, or the like. In addition, the kit may include an antibody that specifically binds to a protein expressed from a gene used as a control group during quantitation. The ELISA may be direct ELISA, indirect ELISA or competitive ELISA.

In addition, the kit may be a kit including essential components required to perform a protein chip. For example, one or more antibodies capable of specifically binding to a protein expressed from the gene may be immobilized and arranged in a high density at a predetermined position on a substrate. In addition, the kit may include an antibody that specifically binds to a protein expressed from a gene used as a control group during quantitation.

### 2. Method for providing information necessary for diagnosis of diffuse type gastric cancer or for determining therapeutic strategy for diffuse type gastric cancer

The present disclosure provides a method for providing information necessary for the diagnosis of diffuse type gastric cancer or for determining a therapeutic strategy for diffuse type gastric cancer.

The method for providing information necessary for the diagnosis of diffuse type gastric cancer or for determining the therapeutic strategy for diffuse type gastric cancer includes measuring an expression level of *SYT11* gene from an isolated biological stomach tissue sample. The information necessary for determining the therapeutic strategy for diffuse type gastric cancer is at least one selected from the group consisting of predicting the prognosis of diffuse type gastric cancer; evaluating the prognosis of diffuse type gastric cancer; and evaluating the efficacy of a *SYT11* targeted therapeutic agent in patients with gastric cancer.

In addition, the method may further include measuring the expression level of at least one gene selected from the group consisting of *ANGPTL2, CACNB1, CCL19, CD34, JAM3, SLIT2, THBS4and VIMENTIN* from the isolated biological stomach tissue sample.

The measuring of the expression level of the gene may be measuring the amount of mRNA of the gene, or measuring the amount of protein expressed from the gene.

The measuring of the amount of mRNA of the gene may be performed using an agent capable of specifically binding to and recognizing the mRNA of the gene or amplifying the amount thereof. As a specific example, the amount of mRNA of the gene may be measured using a primer pair or probe that specifically binds to a base sequence of the mRNA or cDNA produced by reverse transcription of the mRNA, and may be measured using a method such as PCR, RT-PCR, competitive RT-PCR, real-time RT-PCR, Northern blot, microarray, RNase protection assay, base sequencing, and the like, but is not limited thereto and may be measured using any method known to those skilled in the art.

The measuring of the amount of the protein expressed from the gene may be performed by using an agent that specifically binds to and recognizes the protein. As a specific example, the amount of the protein expressed from the gene may be measured using an antibody or aptamer that specifically binds to the protein, and may be measured using a method such as Western blot, protein microarray (protein chip), Enzyme Linked Immunosorbent Assay (ELISA), 2-dimentional electrophoresis, Immunohistochemistry (IHC), immunofluorescence, Co-immunoprecipitation assay, Fluorescence activated cell sorter (FACS), Radioimmunoassay (RIA), Radioimmunodiffusion, Matrix Assisted Laser Desorption/Ionization Time of Flight Mass Spectrometry (MALDI-TOF analysis), etc., but is not limited thereto and may be measured using any method known to those skilled in the art.

According to a specific embodiment of the present disclosure, the *SYT11* gene was expressed at a high level in patients with diffuse type gastric cancer to have a statistically significant relation, while the same result was not observed in intestinal type gastric cancer (FIG. 2). When the expression level of the *SYT11* gene in the biological stomach tissue sample to be analyzed is higher than the expression level of the *SYT11* gene in the normal control group, the subject to be analyzed may be determined to have diffuse type gastric cancer. Therefore, the information necessary for the diagnosis of diffuse type gastric cancer may be provided through the method.

In addition, according to a specific embodiment of the present disclosure, in the case of patients with diffuse type gastric cancer in which the expression level of the *SYT11* gene is high, the survival probability is lower than that of patients with diffuse type gastric cancer in a group classified as having a low expression level, and thus it was confirmed that there was a statistically significant relation between the expression level and the prognosis of diffuse type gastric cancer, and the relation was not significantly shown in patients with intestinal type gastric cancer (FIG. 3). In addition, it was confirmed that even in patients with diffuse type gastric cancer in which the expression level of the *SYT11* gene and the expression level of any one of the *ANGPTL2, CACNB1, CCL19, CD34, JAM3, SLIT2, THBS4,* and *VIMENTIN* were both high, there was a relation with the survival probability (FIG. 5). Therefore, when the expression levels of the genes were measured in samples isolated from patients with diffuse type gastric cancer, it may be predicted that the prognosis will not be bad if the expression levels are high.

The method of the present disclosure may further include comparing the gene expression level measured from the biological stomach tissue sample with a reference value measured from a control sample.

The control sample may be a sample isolated from a group without suffering from diffuse type gastric cancer, a sample isolated from a group of patients with diffuse type gastric cancer, or a group of patients with diffuse type gastric cancer administered with a *SYT11* targeted therapeutic agent, but is not limited thereto and any sample isolated from a group that may be commonly compared in the art for the diagnosis of diffuse type gastric cancer or for determining a therapeutic strategy may be used without limitation.

The reference value measured from the control sample means a reference for values/data obtained by measuring the expression levels of the genes for the control sample, and may be a value that is a reference for separating whether the expression levels of the genes to be expressed in the sample to be measured is overexpressed or underexpressed. The reference value may be an absolute value; a relative value; a value having an upper and/or lower limit; a range of values; a mean value; a middle value; a median value; or a value compared to a specific control or reference value. The reference value may be set according to a method commonly used in the art, and various statistical methods and software for processing statistics may be used, but are not limited thereto.

The diagnosis of the diffuse type gastric cancer may be diagnosing whether a subject to be diagnosed is a normal subject who does not have gastric cancer or a subject who has diffuse type gastric cancer, or diagnosing of whether gastric cancer suffered by the subject to be diagnosed is diffuse type gastric cancer or not. More specifically, the diagnosis of the diffuse type gastric cancer may be diagnosing whether gastric cancer suffered by the subject to be diagnosed is diffuse type gastric cancer or intestinal type gastric cancer.

The prognosis prediction of the diffuse type gastric cancer may be predicting whether the prognosis will be good or bad, which may be predicting the possibility of cure, possibility of improvement, possibility of recurrence, possibility of death, etc. The prognosis prediction may be predicting survival probability of patients with diffuse type gastric cancer, and for example, predicting whether the patients will survive for a relatively long period of time or a relatively short period of time based on a mean or median value of the survival period of time in a group of patients with diffuse type gastric cancer.

The evaluating of the prognosis of the diffuse type gastric cancer is evaluating how the prognosis described in relation to the prognosis prediction is shown in patients with diffuse type gastric cancer, and for example, may be evaluating whether the prognosis of patients who received or not a specific treatment method or drug administration has been improved or worsened over time.

The method of the present disclosure may further include determining that the gastric cancer is diffuse type gastric cancer when the gene expression level measured from the biological stomach tissue sample is higher than the mean or median value of the gene expression level measured from a group without suffering from diffuse type gastric cancer.

In addition, the method may further include determining that the prognosis is poor when the gene expression level measured from the biological stomach tissue sample is higher than the mean or median value of the gene expression level measured from a group without suffering from diffuse type gastric cancer; or the mean or median value of the gene expression level measured from a group of patients with diffuse type gastric cancer.

The evaluating of the efficacy of the *SYT11* targeted therapeutic agent in the gastric cancer patients may be evaluating at least one selected from the group consisting of the necessity of administration of the *SYT11* targeted therapeutic agent; the possibility of expression of drug resistance to the *SYT11* targeted therapeutic agent; sensitivity to the *SYT11* targeted therapeutic agent; and prognosis of treatment with the *SYT11* targeted therapeutic agent.

The evaluating of the efficacy of the *SYT11* targeted therapeutic agent may be one of diagnostic tests for identifying the possibility of occurrence of various results by applying the *SYT11* targeted therapeutic agent to a specific patient with diffuse type gastric cancer, and may be referred to as a companion diagnostic.

The *SYT11* targeted therapeutic agent may be, for example, a *SYT11* inhibitor, and is used in the meaning of collectively referring to all agents of reducing the expression or activity of *SYT11.* Specifically, the *SYT11* targeted therapeutic agent may include all agents of reducing an expression level of *SYT11* in a transcription, mRNA level or translation level by a method such as affecting the reduction of the expression of *SYT11,* directly acting on *SYT11,* or indirectly acting on a ligand thereof, or reducing the activity of *SYT11* by inhibiting the activity of *SYT11.*

The *SYT11* inhibitor is able to be used without limitation in the form thereof, such as compounds, nucleic acids, peptides, virus, vectors containing the nucleic acids, or the like capable of inhibiting the expression of *SYT11* or the activity by targeting *SYT11.* The *SYT11* inhibitor is not limited thereto, but includes siRNA or shRNA that degrades mRNA of the *SYT11* gene, and antisense oligonucleotide that reduces the expression of the *SYT11* protein. Further, the *SY711* inhibitor that binds to the *SYT11* protein to inhibit the function may include an aptamer or a low molecular compound.

In a specific embodiment of the present disclosure, it was confirmed that the expression level of the *SYT11* gene and the expression level of the gene selected from the group consisting of *ANGPTL2, CACNB1, CCL19, CD34, JAM3, SLIT2, THBS4,* and *VIMENTIN* in patients with diffuse type gastric cancer showed the same pattern to have a statistically significant relation. In addition, it was confirmed that the expression level of the *SYT11* gene and the expression level of the gene selected from the group consisting of *ANGPTL2, CACNB1, CCL19, CD34, JAM3, SLIT2, THBS4,* and *VIMENTIN* are closely related to the prognosis of patients with diffuse type gastric cancer to have the same pattern. Therefore, when administering a therapeutic agent for diffuse type gastric cancer targeting *SYT11,* the expression level of the gene selected from the group consisting of *ANGPTL2, CACNB1, CCL19, CD34, JAM3, SLIT2, THBS4,* and *VIMENTIN* is measured, and thus the measurement result at the expression level may be used for evaluating the possibility of expressing drug resistance to the *SYT11* targeted therapeutic agent; sensitivity to the *SYT11* targeted therapeutic agent; and prognosis of treatment with the *SYT11* targeted therapeutic agent.

The necessity of the administration is to determine whether to administer the therapeutic agent to a subject who is able to obtain or expected to obtain a therapeutic effect from the therapeutic agent.

The method may further include determining that the subject providing the sample is likely to correspond to at least one of the following cases, when the expression level of a gene selected from the group consisting of *ANGPTL2, CACNB1, CCL19, CD34, JAM3, SLIT2, THBS4* and *VIMENTIN,* measured from a biological stomach tissue sample isolated from a patient with a diffuse type, is higher than a reference value: Necessity of administration of a *SYT11* targeted therapeutic agent; low possibility of expression of drug resistance to the *SYT11* targeted therapeutic agent; high sensitivity to the *SYT11* targeted therapeutic agent; or good prognosis for treatment with the *SYT11* targeted therapeutic agent.

In one specific embodiment of the present disclosure, it is possible to confirm that the *SYT11* gene is expressed in a biological stomach sample obtained from patients with diffuse type gastric cancer, to measure the expression level of at least one gene selected from the group consisting of *ANGPTL2, CACNB1, CCL19, CD34, JAM3, SLIT2, THBS4,* and *VIMENTIN,* and then determine whether to prescribe a *SYT11* targeted therapeutic agent. Specifically, when the expression levels of the genes of the patients are measured to be higher than or equal to a reference value, the patients are expected to have high sensitivity to the *SYT11* targeted therapeutic agent or low resistance to the *SYT11* targeted therapeutic agent, thereby providing information necessary for determining whether to prescribe the *SYT11* targeted therapeutic agent.

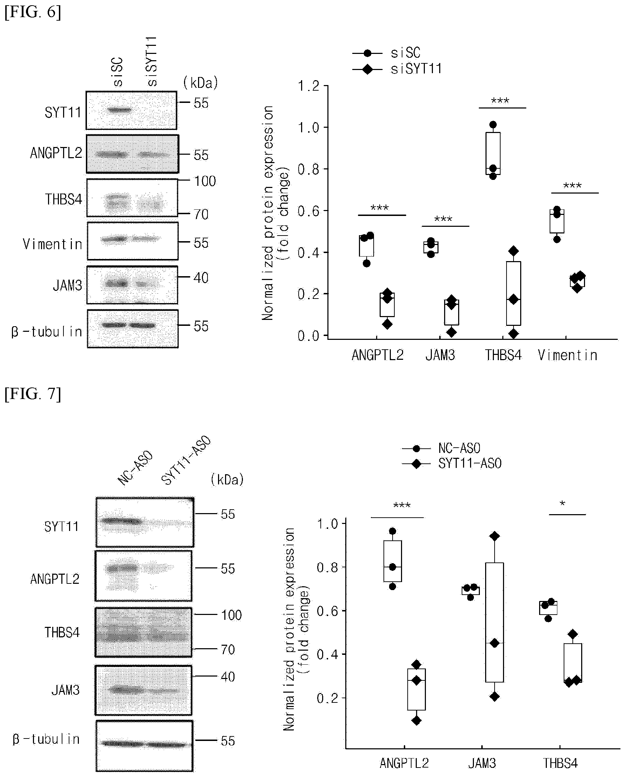

In addition, according to a specific embodiment of the present disclosure, when a gastric cancer cell line was treated with a *SYT11* targeted therapeutic agent such as siSYT11 or SYT11-ASO that targets SYT11 and serves to inhibit the expression or activity thereof, it was confirmed that not only the amount of SYT11 protein but also the amounts of ANGPL2, THBS4, VIMENTIN, and JAM3 proteins were decreased in the cells together (FIGS. 6 and 7). Accordingly, it may be seen that the markers inhibit and regulate the expression or activity thereof together according to the inhibition of *SYT11,* and sensitivity, resistance, etc. to the *SYT11* targeted therapeutic agent may be confirmed by monitoring the markers when administering the *SYT11* targeted therapeutic agent, and thus the markers may be used to evaluate the efficacy of the *SYT11* targeted therapeutic agent.

Hereinafter, the present disclosure will be described in detail by Examples and Experimental Examples.

However, the following Examples and Experimental Examples are just illustrative of the present disclosure, and the contents of the present disclosure are not limited to the following Examples and Experimental Examples.

### [Example 1]

### Confirmation of relation between expression level of SYT11 gene and diffuse type gastric cancer

The relation between an expression level of *SYT11* gene and gastric cancer was confirmed by measuring and comparing the expression levels of the *SYT11* gene expressed from gastric cancer tissues and normal stomach tissues.

Cancer and normal tissues were purchased from Biomax (US), and immunohistochemistry was performed on 70 gastric cancer tissues and 10 normal tissues to confirm the expression levels of *SYT11.*

Specifically, the tissue was immersed in a xylene solution for 10 minutes to remove paraffin from each tissue, and then immersed in 100%, 95%, 80%, and 70% ethanol solutions for 5 minutes in sequence to remove the remaining xylene, and then rinsed with water. The tissue slide was immersed in a 3% hydrogen peroxide (H₂O₂) solution for 10 minutes and then immersed and rinsed in water for 15 minutes. After boiling a 0.01 M sodium citrate solution at 95°C, the tissue slide was placed in the solution, left for 10 minutes, and then cooled at room temperature for 30 minutes. The tissue slide was rinsed with water for 10 minutes, and then immersed in a buffer solution (phosphate buffered saline) for 10 minutes to prepare a sample, and the prepared sample reacted sequentially with an anti-*SY711* antibody, a biotin-conjugated secondary antibody (Vector Lab, USA), and Abidin+Biotinylated horseradish peroxidase (Vector Lab, USA), and then the expression level of *SYT11* protein was confirmed using a DAB peroxidase substrate (Vector Lab, USA). Then, to distinguish the nuclear portion of the tissue, the tissue slide reacted with a hematoxylin solution for 5 minutes, rinsed with water, and then immersed in 0.1% hydrogen chloride (HCl) and 0.1% ammonia (NH₄OH) solutions in sequence, and then rinsed with water. The tissue slide was sequentially immersed in 70%, 85%, 95%, and 100% ethanol for 5 minutes each, reacted in a xylene solution for 5 minutes, and then dried.

According to the color development of *SYT11* gene measured by immunohistochemistry, the tissues were classified into a group with a low expression level *(SYT11-low)* and a group with a high expression level (*SY711*-high) of *SYT11* gene based on the median or mean value thereof. As a result, the expression level of the *SYT11* gene was found to be high in only 30% of normal stomach tissue, but the expression level of the *SYT11* gene was found to be high in 64.3% of patients in the stomach tissue of gastric cancer patients. Therefore, it was confirmed that the expression level of the *SYT11* gene was correlated with the occurrence of gastric cancer (FIG. 1).

In addition, the 70 gastric cancer tissues were histologically divided into 30 intestinal type gastric cancer tissues and 40 diffuse type gastric cancer tissues, and the expression level of the *SYT11* gene was measured by the degree of color development, respectively, and similarly, the tissues were classified into a group with a low expression level (*SY711*-low) and a group with a high expression level (*SY711-*high) of the *SYT11* gene. As a result, in the case of the intestinal type gastric cancer tissues, the expression level of the *SYT11* gene was found to be high in 46.7%, but in the case of stomach tissues of patients with diffuse type gastric cancer, the expression level of the *SYT11* gene was found to be high in 77.5% of patients. Therefore, it was confirmed that the expression level of the *SYT11* gene was correlated with diffuse type gastric cancer (FIG. 1).

### [Example 2]

### Comparison of difference in SYT11 gene expression levels between diffuse type gastric cancer and intestinal gastric cancer

Microarray results were analyzed for 239 patients with intestinal type gastric cancer and 185 patients with diffuse type gastric cancer provided from the Yonsei University Severance Hospital. In the microarray results, after analyzing a receiver operating characteristic curve according to the *SYT11* expression level, a gastric cancer patient group was classified into groups with high or low *SYT11* expression levels based on the median or mean value thereof. In addition, it was also confirmed whether gastric cancer was stem-like gastric cancer or non-stem-like gastric cancer.

As a result, in the case of intestinal type gastric cancer, 77 of 239 patients were found to have high *SYT11* gene expression levels, and among them, 27 of 31 stem-like patients were analyzed to have high *SYT11* gene expression levels. On the other hand, in the case of diffuse type gastric cancer, 100 of 185 patients were found to have high *SYT11* gene expression levels, and among them, 67 of 80 stem-like patients were found to have high *SYT11* gene expression levels (FIG. 2).

Through the results, in addition to reconfirming that the expression of the *SYT11* gene was related to stem-like gastric cancer as in previous studies, it was newly confirmed that the expression of the *SYT11* gene was found to be low in intestinal type gastric cancer and high in diffuse type gastric cancer, and thus it was confirmed that the expression level of the *SYT11* gene was related to diffuse type gastric cancer and thus the *SYT11* gene may be used as a diagnostic marker for diffuse type gastric cancer.

### [Example 3]

### Confirmation of relation between expression level of SYT11 gene and prognosis in patients with diffuse type gastric cancer

Based on clinical data of 424 gastric cancer patients according to Example 2 above, a relation between the survival probability of gastric cancer patients and the expression level of the *SYT11* gene was analyzed.

Specifically, 239 patients with intestinal type gastric cancer and 185 patients with diffuse type gastric cancer were classified, and through the microarray results, these patients were divided into groups with high and low expression levels based on the expression levels of *SYT11* and *SYT4* genes. In addition, a relation between the gene expression levels and the prognosis of gastric cancer patients was confirmed by comparing survival probability data of each group.

As a result, as may be seen in FIG. 3, in the case of 239 patients with intestinal type gastric cancer, there was no statistically significant difference in survival probability between the groups with high and low expression levels of the *SYT11* gene (p = 0.3363). On the other hand, in the case of 185 patients with diffuse type gastric cancer, it was confirmed that the group with the high expression level of the *SYT11* gene showed significantly low survival probability (p = 0.0067) to have a close correlation between the expression level of the *SYT11* gene and the prognosis of patients with diffuse type gastric cancer. In addition, in the result of a control group that confirmed the relation between the expression level of *SYT4gene,* which belonged to the same family as the *SYT11* gene, and the survival probability, it was confirmed that the prognosis of diffuse type patients and the expression level of the *SYT4* gene were not significantly related (p = 0.1618), and it was confirmed that diffuse type gastric cancer was related only to the expression level of the *SYT11* gene.

### [Example 4]

### Discovery of 8 companion diagnostic markers and confirmation of relation with diffuse type gastric cancer

Through Examples above, it was confirmed that the expression level of the *SYT11* gene was high in patients with diffuse type gastric cancer, and that diffuse type patients with the high expression level of the *SYT11* gene had a poor prognosis. Accordingly, by identifying genes whose expression levels were regulated in the same tendency as the *SYT11* gene, there were discovered eight novel markers that were likely to be used as companion diagnostic markers (FIG. 4).

Specifically, based on the microarray results of gastric cancer patients, the receiver operating characteristic curves were analyzed according to the expression levels of *ANGPTL2, CACNB1, CCL19, CD34, JAM3, SLIT2, THBS4,* and *VIMENTIN* genes, and then the patients were classified into groups with high and low expression levels based on the median and mean values thereof according to an expression level of each gene. In addition, the patient survival rate according to the expression levels of *SYT11* and each gene was represented by a Kaplan-Meier graph.

As may be seen in FIG. 4, in patients with the high expression level of the *SYT11* gene, the expression levels of *ANGPTL2, CACNB1, CCL19, CD34, JAM3, SLIT2, THBS4,* and *VIMENTIN,* 8 genes also increased in proportion to the expression level of the *SYT11* gene, and had a statistically significant relation.

In addition, the survival probability was confirmed in patients with intestinal type gastric cancer and patients with diffuse type gastric cancer when the expression levels of both the *SYT11* gene and *ANGPL2gene* were high (SYT11(H)-ANGPL2(H)) or low (SYT11(L)-ANGPL2(L)). In addition, a relation with the survival probability was compared in the same manner even for seven other genetic markers other than the *ANGPL2* gene. As a result, diffuse type patients with high expression levels of both the *SYT11* gene and each of the eight genes had low survival probability and thus poor prognosis and a statistically significant result was shown. On the other hand, in the case of intestinal type gastric cancer, it was confirmed that there was no significant relation between the survival probability and the expression levels of all of the eight markers, and it was found that the eight genetic markers were correlated with the expression level of the *SYT11* gene only in diffuse type gastric cancer (FIG. 5).

### [Example 5]

### Confirmation of expression levels of companion diagnostic markers according to treatment of SYT11 targeted therapeutic agent

siSYT11 (5'-GCAGAAAGCGCAUUGCCAA(dTdT)-3', d referred to deoxyribonucleic acid), a type of siRNA as an *SYT11* targeted therapeutic agent capable of binding to mRNA of the *SYT11* gene to inhibit the expression thereof, was transfected into SNU484 cells as a gastric cancer cell line. As a control group, siScramble (5'-CCUACGCCACCAAUUUCGU(dTdT)-3', d referred to deoxyribonucleic acid) was transfected. After 48 hours of transfection, proteins were extracted using an RIPA buffer and separated by electrophoresis (SDS-PAGE) on a polyacrylamide gel. Thereafter, Western blotting was performed using each antibody of the companion diagnostic marker genes discovered through Example 4.

As a result, it was confirmed that the amounts of ANGPL2, THBS4, VIMENTIN2, and JAM3 were reduced as the amount of *SYT11* protein was reduced in the cells using siSYT11 as a SYT11-targeting therapeutic agent (FIG. 6).

In addition, SYT11-ASO (5'-mU*mU*G*G*C*A*A*T*G*C*G*C*T*T*T*C*T*mG*mC-3', in which m represented methylation and * represented phosphorothioate), which was a type of antisense oligonucleotide as another type of *SYT11* targeted therapeutic agent, was transfected into SNU484 cells as a gastric cancer cell line. As a control group, NC-ASO (5'-mC*mC*T*A*C*G*C*C*A*C*C*A*A*T*T*T*C*mG*mU-3', in which m represented methylation and * represented phosphorothioate) was transfected. After 48 hours of transfection, proteins were extracted using an RIPA buffer and separated by electrophoresis (SDS-PAGE) on a polyacrylamide gel. Thereafter, Western blotting was performed using each antibody of the companion diagnostic marker genes discovered through Example 4.

As a result, it was confirmed that the amounts of ANGPL2, THBS4, VIMENTIN2, and JAM3 were reduced as the amount of SYT11 protein was reduced in the cells using SYT11-ASO, similarly to when siSYT11 was used (FIG. 7).

Based on the results, the *ANGPL2, THBS4, VIMENTIN2,* and *JAM3genes* may be expected as downstream genes of *SYT11* regulated by *SYT11,* and it may be expected that the expression levels of the genes of Example 4, which are significantly related to the expression level of the *SYT11* gene, will be regulated together when the expression of *SYT11* has been regulated. Therefore, when using a targeted therapeutic agent that reduces the *SYT11* expression or inhibits the *SYT11* activity, the expression levels of *ANGPL2, THBS4, VIMENTIN,* and *JAM3are* measured to determine whether the expression levels are reduced, thereby monitoring the therapeutic effect, resistance, and the like of the *SYT11* targeted therapeutic agent. Accordingly, it is expected that these genes may be utilized as companion diagnostic markers for the *SYT11* targeted therapeutic agent.

### [Example 6]

### Conclusion

When combining the results of Examples above, it was confirmed that the *SYT11* gene had a high expression level in patients with diffuse type gastric cancer, and that the *SYT11* gene may be used as a marker for the diagnosis of diffuse type gastric cancer. In addition, it was confirmed that the expression level of the *SYT11* gene was related to the prognosis of diffuse type patients, and thus, when the expression level of the *SYT11* gene was high, it may be predicted that the prognosis of diffuse type patients will be poor. In addition, in patients with diffuse type gastric cancer, it was confirmed that the expression level of the *SYT11* gene and the expression levels of eight genes *ANGPTL2, CACNB1, CCL19, CD34, JAM3, SLIT2, THBS4,* and *VIMENTIN* showed similar trends and were found to be related, and thus also related to the prognosis of patients with diffuse type gastric cancer. Therefore, it was confirmed that the eight genes may also be used as markers for predicting the prognosis of diffuse type gastric cancer.

Furthermore, previous studies have found that *SYT11* may be a target for treating gastric cancer, and it was experimentally confirmed that when siSYT11 or SYT11-ASO capable of reducing the amount of *SYT11* was treated to a gastric cancer cell line, the amount of the marker genes was also reduced. Therefore, in relation to the treatment of diffuse type gastric cancer targeting *SYT11,* it was confirmed that the eight markers may be used as companion diagnostic markers to obtain information such as whether SYT11-targeted treatment is necessary, prediction of sensitivity to administration of the SYT11-targeted therapeutic agent, and prognosis after therapeutic agent administration.

As described above, while the present disclosure has been described in detail with reference to the described examples, it is apparent to those skilled in the art that various modifications and changes may be made within the technical idea of the present disclosure, and it is natural that these modifications and changes belong to the appended claims.

## Claims

1. A composition for the diagnosis or prognosis of diffuse type gastric cancer, comprising an agent for measuring an expression level of *SYT11* gene.

2. The composition for the diagnosis or prognosis of diffuse type gastric cancer of claim 1, further comprising:
at least one agent for measuring an expression level of a gene selected from the group consisting of *ANGPTL2, CACNB1, CCL19, CD34, JAM3, SLIT2, THBS4,* and *VIMENTIN.*

3. The composition for the diagnosis or prognosis of diffuse type gastric cancer of claim 1 or 2, wherein the agent for measuring the expression level of the gene is at least one of an agent for measuring the amount of mRNA of the gene and an agent for measuring the amount of protein expressed from the gene.

4. The composition for the diagnosis or prognosis of diffuse type gastric cancer of claim 3, wherein the agent for measuring the amount of mRNA of the gene comprises a primer pair or probe that specifically binds to mRNA or cDNA of the gene.

5. The composition for the diagnosis or prognosis of diffuse type gastric cancer of claim 3, wherein the agent for measuring the amount of the protein expressed from the gene comprises an antibody or aptamer specific for the protein expressed from the gene.

6. A kit for the diagnosis or prognosis of diffuse type gastric cancer, comprising the composition according to any one of claims 1 to 5.

7. A method for providing information necessary for the diagnosis of diffuse type gastric cancer or for determining a therapeutic strategy for diffuse type gastric cancer, comprising:
measuring an expression level of *SYT11* gene from an isolated biological stomach tissue sample,
wherein the information necessary for determining the therapeutic strategy for diffuse type gastric cancer is at least one selected from the group consisting of predicting the prognosis of diffuse type gastric cancer; evaluating the prognosis of diffuse type gastric cancer; and evaluating the efficacy of a *SYT11* targeted therapeutic agent in patients with gastric cancer.

8. The method for providing information necessary for the diagnosis of diffuse type gastric cancer or for determining a therapeutic strategy for diffuse type gastric cancer of claim 7, further comprising:
measuring the expression level of at least one gene selected from the group consisting of *ANGPTL2, CACNB1, CCL19, CD34, JAM3, SLIT2, THBS4* and *VIMENTIN* from the isolated biological stomach tissue sample.

9. The method for providing information necessary for the diagnosis of diffuse type gastric cancer or for determining a therapeutic strategy for diffuse type gastric cancer of claim 7 or 8, further comprising:
comparing the gene expression level measured from the biological stomach tissue sample with a reference value measured from a control sample.

10. The method for providing information necessary for the diagnosis of diffuse type gastric cancer or for determining a therapeutic strategy for diffuse type gastric cancer of claim 9, further comprising:
determining that the gastric cancer is diffuse type gastric cancer when the gene expression level measured from the biological stomach tissue sample is higher than a mean or median value of the gene expression level measured from a group without suffering from diffuse type gastric cancer.

11. The method for providing information necessary for the diagnosis of diffuse type gastric cancer or for determining a therapeutic strategy for diffuse type gastric cancer of claim 9, further comprising:
determining that the prognosis is poor when the gene expression level measured from the biological stomach tissue sample is higher than a mean or median value of the gene expression level measured from a group without suffering from diffuse type gastric cancer or a mean or median value of the gene expression level measured from a group of patients with diffuse type gastric cancer.

12. The method for providing information necessary for the diagnosis of diffuse type gastric cancer or for determining a therapeutic strategy for diffuse type gastric cancer of claim 7, wherein the evaluating of the efficacy of the *SYT11* targeted therapeutic agent in the gastric cancer patients is evaluating at least one selected from the group consisting of the necessity of administering the *SYT11* targeted therapeutic agent; the possibility of expressing drug resistance to the *SYT11* targeted therapeutic agent; the sensitivity to the *SYT11* targeted therapeutic agent; and the prognosis of treatment with the *SYT11* targeted therapeutic agent.
